# NEUE EUROPÄISCHE PATENTSCHRIFT

(11) **EP 0 892 778 B2**
(45) Veröffentlichungstag und Bekanntmachung des Hinweises auf die Entscheidung über den Einspruch: **01.04.2015**
(45) Hinweis auf die Patenterteilung: 25.07.2001
(21) Anmeldenummer: 97918107.0
(22) Anmeldetag: 04.04.1997
(51) Int. Cl.: C07C 209/48, C07C 253/30, C07C 211/12, C07C 255/24

(54) **VERFAHREN ZUR GLEICHZEITIGEN HERSTELLUNG VON 6-AMINOCAPRONITRIL UND HEXAMETHYLENDIAMIN**
METHOD OF SIMULTANEOUSLY PRODUCING 6-AMINOCAPRONITRILE AND HEXAMETHYLENEDIAMINE
PROCEDE PERMETTANT DE PRODUIRE SIMULTANEMENT DU 6-AMINOCAPRONITRILE ET DE L'HEXAMETHYLENEDIAMINE

(30) Priorität: 10.04.1996 DE 19614154
(43) Veröffentlichungstag der Anmeldung: 27.01.1999
(73) Patentinhaber: BASF SE, 67056 Ludwigshafen (DE)
(72) Erfinder: SCHNURR, Werner, D-67273 Herxheim (DE); VOIT, Guido, D-69198 Schriesheim (DE); FLICK, Klemens, D-76863 Herxheim (DE); MELDER, Johann-Peter, D-67141 Neuhofen (DE); FISCHER, Rolf, D-69121 Heidelberg (DE); HARDER, Wolfgang, D-69469 Weinheim (DE)
(74) Vertreter: Féaux de Lacroix, Stefan
(86) Internationale Anmeldenummer: PCT/EP1997/001685
(87) Internationale Veröffentlichungsnummer: WO 1997/037964

(56) Entgegenhaltungen:
- EP-A- 0 061 042
- WO-A-92/21650
- US-A- 2 620 346
- US-A- 3 794 602
- US-A- 4 361 495
- JOURNAL OF CATALYSIS, Bd. 143, 1993, Seiten 187-200, XP002034544 VERHAAK ET AL.: "The Deactivation of Nickel Catalysts in the Hydrogenation of Acetonitrile" in der Anmeldung erwähnt
- JP-B-46008283 (Engl. Übersetzung)
- A.F. HOLLEMAN ET AL.: 'Lehrbuch der Anorganischen Chemie', Bd. 81-90, 1976, WALTER DE GRUYTER, BERLIN - NEW YORK Seiten 184 - 185
- M. GUISNER ET AL.: 'Selective hydrogenation of dinitriles to aminonitriles on Raney catalysts', Bd. 78, 1993, ELSEVIER SCIENCE PUBLISHERS B.V. Seiten 283 - 290
- J. R. ANDERSON ET AL.: 'Catalysis Science & Technology', Bd. 1, 1981, SPRINGER-VERLAG, BERLIN HEIDELBERG NEW YORK Seiten 200 - 201
- A. B. STILES: 'Catalyst Supports and Supported Catalysts, Theoretical and Applied Concepts', 1997, BUTTERWORTHS, BOSTON Seite 244
- M.J.F.M. VERHAAK ET AL.: 'The Deactivation of Nickel Catalysts in the Hydrogenation of Acetonitrile' JOURNAL OF CATALYSIS Bd. 143, 1993, Seiten 187 - 200

## Beschreibung

Die vorliegende Erfindung betrifft ein verbessertes Verfahren zur gleichzeitigen Herstellung von 6-Aminocapronitril ("ACN") und Hexamethylendiamin ("HMD") durch Behandlung von Adipodinitril ("ADN") mit Wasserstoff in Gegenwart eines Nickel-haltigen Katalysators bei Temperaturen von nicht unterhalb der Raumtemperatur und erhöhtem Wasserstoff-Partialdruck in Suspension oder in einem Festbettreaktor in Riesel- oder Sumpffahrweise in Gegenwart eines Lösungsmittels.

Die Hydrierung von ADN zu 6-Aminocapronitril in Gegenwart von Lösungsmitteln, insbesondere Ammoniak, und Nickel-haltigen Katalysatoren ist beispielsweise in der US-A 2,762,835, der US 2,208,598 und der WO 92/21650 ausführlich beschrieben, so daß nähere Angaben hierzu entbehrlich sind.

Die bei der Hydrierung von ADN eingesetzten Nickel-haltigen Katalysatoren verlieren in Langzeitversuchen an Aktivität und müssen daher nach Absinken der Aktivität unter einen bestimmten Wert durch neue Katalysatoren ersetzt werden.

Nickel-haltige Katalysatoren werden in der Technik als weit verbreitete Katalysatoren zum Steam-Reforming, zur Methanisierung und zur Hydrierung funktioneller Gruppen wie CO-Doppelbindungen, C-C-Mehrfachbindungen oder Nitrilgruppen eingesetzt. Bei vielen der vorher genannten Anwendungsgebiete bilden sich auf der aktiven Katalysatoroberfläche C-haltige Ablagerungen, die eine mehr oder weniger schnelle Desaktivierung des Katalysators bewirken. Die Bildung von C-haltigen Ablagerungen beim Steam-Reforming und die Entfernung dieser Schichten durch Reaktion mit Sauerstoff, Wasserstoff, Dampf oder Kohlendioxid wird in Trimm, Catal. Rev.-Sci. Eng., 16(2), 155-187 (1977) beschrieben. Meßbare Reaktions geschwindigkeiten werden mit Wasserstoff erst bei Temperaturen über 550°C erzielt.

Die Regenerierung von mit Kohlenstoff-haltigen Ablagerungen belegten Katalysatoren erfolgt in der Regel durch Abbrennen der organischen Beläge mit Stickstoff/Luft-Gemischen. Diese Methode kann jedoch nur bei solchen Katalysatoren angewandt werden, die bei der Umsetzung mit Luft mechanisch stabil bleiben. Bei Trägerkatalysatoren, die ein stabiles Gerüst aus oxidischem Material, wie SiO₂, Al₂O₃, TiO₂ aufweisen, kann diese Regenerierungsmethode mit Erfolg eingesetzt werden. So wird in GB-A 2,284,163 die Regenerierung eines Trägerkatalysators mit Pt, Pd, Ru, Rh, etc. oder Nickel durch eine Behandlung eines mindestens Chlor und Sauerstoff enthaltenden Gases beschrieben.

Katalysatoren mit sehr hohen Metallgehalten nehmen durch Abbrennen der organischen Beläge durch Luft Schaden und verändern ihre mechanischen Eigenschaften (siehe EP-A 61,042).

Aus EP-A 61,042 ist auch bekannt, daß Nickel-haltige Katalysatoren mit maximal 50 Gew.-% Nickel für die Hydrierung von Butindiol zu Butandiol durch eine Wasserstoffbehandlung bei Temperaturen zwischen 200 und 500°C, vorzugsweise bei Temperaturen über 275°C regeneriert werden können.

Ebenfalls eine Regenerierung mit Wasserstoff wird in der US-A 5,310,713 für einen Alkylierungskatalysator, der Nickel enthalten kann, beschrieben, wobei die Regenerierung mit Wasserstoff jedoch in Gegenwart eines flüssigen Alkans und einer Chlorid-Quelle durchgeführt wird.

Es ist aus Journal of Catalysis 143 (1993) S. 187-200 bekannt, daß man einen Nickel-Katalysator (25 Gew.-% Ni auf SiO₂), der für die Hydrierung von Acetonitril in der Gasphase eingesetzt wird, durch Behandlung mit Wasserstoff bei Temperaturen über 200°C regenerieren kann.

Aus dem genannten Stand der Technik ist nicht zu entnehmen, ob man auch Nickel-haltige Katalysatoren regenerieren kann, die bei der Hydrierung von höhersiedenden Dinitrilen, insbesondere Adipodinitril, eingesetzt werden. Denn gerade bei bifunktionellen Verbindungen wie Dinitrilen können unter Reaktionsbedingungen Oligomere gebildet werden, die bei der Regenerierung zu Problemen führen.

Der vorliegenden Erfindung lag daher die Aufgabe zugrunde, ein ) Verfahren zur Verfügung zu stellen, das es gestattet, die bei der Hydrierung von ADN zu ACN und HMD eingesetzten Nickel-haltigen Katalysatoren mit einfachen Mitteln zu regenerieren, so daß es zu keinen langen Stillstandzeiten während der Regenerierung der Katalysatoren kommt. Insbesondere war es das Ziel, die Katalysatoraktivität hinsichtlich Umsatz und Selektivität bei der Hydrierung von ADN möglichst wieder auf das Niveau des ungebrauchten Katalysators anzuheben.

Demgemäß wurde ein Verfahren zur gleichzeitigen Herstellung von 6-Aminocapronitril ("ACN") und Hexamethylendiamin ("HMD") durch Behandlung von Adipodinitril ("ADN") mit Wasserstoff in Gegenwart eines Nickel-haltigen Katalysators bei Temperaturen von nicht unterhalb der Raumtemperatur und erhöhtem Wasserstoff-Partialdruck in Suspension oder in einem Festbettreaktor in Riesel- oder Sumpffahrweise in Gegenwart eines Lösungsmittels gefunden, indem man nach Absinken des Umsatzes, bezogen auf ADN, und/oder der Selektivität, bezogen auf ACN, unter einen definierten Wert,
(a) die Behandlung von ADN mit Wasserstoff unterbricht, indem man die Zufuhr von ADN und dem gewünschtenfalls vorhandenen Lösungsmittel stoppt,
(b) den Katalysator bei einer Temperatur im Bereich von 180 bis 270°C mit Wasserstoff behandelt, wobei man den Wasserstoffdruck im Bereich von 0,1 bis 30 MPa und die Behandlungszeit im Bereich von 2 bis 48h wählt, und
(c) anschließend die Hydrierung von ADN mit dem nach Stufe (b) behandelten Katalysator fortsetzt.

Als Nickel-Katalysatoren kann man übliche Raney-Nickel-Katalysatoren (als Festbett- oder Suspensionskatalysatoren) oder Trägerkatalysatoren verwenden. Raney-Nickel-Katalysatoren sind bekannt und käuflich zu erwerben oder in bekannter Weise aus einer Nikkel-Aluminium-Legierung durch Behandlung mit einer Base wie Natronlauge herstellbar. Als Träger kann man üblicherweise Aluminiumoxid, Siliciumdioxid, Aktivkohlen, Titandioxid und Zirkondioxid verwenden. Bei geträgerten Katalysatoren wählt man üblicherweise einen Nickelgehalt im Bereich von 3 bis 95, bevorzugt von 20 bis 95, besonders bevorzugt von 50 bis 95 Gew.-%, bezogen auf die Gesamtmasse an Nickel und Träger.

Die Katalysatoren kann man auch, wenn gewünscht, mit Metallen der Gruppe VIB (Cr, Mo, W) und VIII des Periodensystems der Elemente (Fe, Ru, Os, Co, Rh, Ir, Pd, Pt) sowie mit Kupfer, Rhenium oder Mangan modifizieren, wobei der Nickel-Gehalt im Katalysator im allgemeinen im Bereich von 50 bis 99,9, bevorzugt von 80 bis 99 Gew. -%, bezogen auf die aktiven Komponenten (Nickel + Modifizierungsmittel), beträgt.

Des weiteren kann man die Katalysatoren mit einer Verbindung auf der Basis eines Alkalimetalles oder Erdalkalimetalles wie Lithium, Natrium, Kalium, Rubidium, Cäsium, Magnesium, Calcium, Strontium und Barium, insbesondere Cäsium, modifizieren. Üblicherweise wählt man ein Gewichtsverhältnis im Bereich von 0 bis 5, bevorzugt von 0,1 bis 3 Gew.-% Alkalimetall bzw. Erdalkalimetall zu Nickel.

Die erfindungsgemäß einsetzbaren Ni-Katalysatoren können auf unterschiedliche Art und Weise hergestellt werden. Die Herstellung von geträgerten Ni-Katalysatoren erfolgt üblicherweise durch Tränkung eines keramischen Trägers mit einer wäßrigen oder organischen Lösung eines Ni-Salzes und gewünschtenfalls des Modifizierungsmittels, anschließende Trocknung und Calcinierung in an sich bekannter Weise. Die Löslichkeit der Salze und das Porenvolumen des Trägers begrenzt nach bisherigen Beobachtungen die Menge an Nickel, die durch einen Tränkschritt aufgebracht werden kann, so daß gewünschtenfalls die Tränkprozedur mehrmals wiederholt werden muß, wobei in der Regel nach jedem Tränkschritt der Katalysator getrocknet und calziniert wird, um den gewünschten Gehalt an Nickel auf dem Katalysator zu erhalten. Es ist auch möglich, Nickel durch Ausfällen einer schwerlöslichen Nickel-Verbindung wie die entsprechende Hydroxid- oder Carbonat-Verbindung auf einen in der Fällungslösung suspendierten Träger aufzubringen. Die durch die Fällung erhaltenen Niederschläge können üblicherweise nach Filtration oder Sprühtrocknung nach an sich bekannten Methoden verformt werden.

Die Hydrierungen führt man erfindungsgenäß in Sumpf-, Riesel- oder Suspensionsfahrweise durch.

Führt man die Umsetzung in einer Suspension durch, wählt man üblicherweise Temperaturen im Bereich von 40 bis 150, vorzugsweise von 50 bis 100, besonders vorzugsweise von 60 bis 90°C; den Druck wählt man im allgemeinen im Bereich von 2 bis 20, vorzugsweise von 3 bis 10, besonders bevorzugt von 4 bis 9 MPa. Die Verweilzeiten sind im wesentlichen von der gewünschten Ausbeute, Selektivität und dem gewünschten Umsatz abhängig; üblicherweise wählt man die Verweilzeit so, daß ein Maximum an Ausbeute erreicht wird, beispielsweise im Bereich von 50 bis 275, vorzugsweise von 70 bis 200 min.

Bei der Suspensionsfahrweise kann man als Lösungsmittel bevorzugt Ammoniak, Amine, Diamine und Triamine mit 1 bis 6 C-Atomen wie Trimethylamin, Triethylamin, Tripropylamin und Tributylamin oder Alkohole, insbesondere Methanol und Ethanol, besonders bevorzugt Ammoniak, einsetzen. Zweckmäßig wählt man eine Dinitrilkonzentration im Bereich von 10 bis 90, vorzugsweise von 30 bis 80, besonders vorzugsweise von 40 bis 70 Gew.-%, bezogen auf die Summe von Dinitril und Lösungsmittel.

Die Menge an Katalysator wählt man im allgemeinen so, daß die Katalysator-Menge im Bereich von 1 bis 50, bevorzugt von 5 bis 20 Gew.-%, bezogen auf die eingesetzte Menge an Dinitril, beträgt.

Die Suspensionshydrierung kann man diskontinuierlich oder, bevorzugt kontinuierlich, in der Regel in der Flüssigphase durchführen.

Man kann die Hydrierung auch diskontinuierlich oder kontinuierlich in einem Festbettreaktor in Riesel- oder Sumpffahrweise durchführen, wobei man üblicherweise eine Temperatur im Bereich von 20 bis 150, vorzugsweise von 30 bis 90°C und einen Druck in der Regel im Bereich von 2 bis 30, vorzugsweise von 3 bis 20 MPa wählt. Man führt die Hydrierung in Gegenwart eines Lösungsmittels, bevorzugt Ammoniak, Amine, Diamine und Triamine mit 1 bis 6 C-Atomen wie Trimethylamin, Triethylamin, Tripropylamin und Tributylamin oder Alkohol, bevorzugt Methanol und Ethanol, besonders bevorzugt Ammoniak, durch. In einer bevorzugten Ausführungsform wählt man einen Gehalt an Ammoniak im Bereich von 0,5 bis 10, bevorzugt von 1 bis 6 g pro g Adipodinitril. Bevorzugt wählt man dabei eine Katalysatorbelastung im Bereich von 0,1 bis 2,0, vorzugsweise von 0,3 bis 1,0 kg Adipodinitril/l*h. Auch hier kann man durch Veränderung der Verweilzeit den Umsatz und damit die Selektivität gezielt einstellen.

Die Hydrierung kann man in einem üblichen hierfür geeigneten Reaktor durchführen.

In einer bevorzugten Ausführungsform führt man die Hydrierung von ADN in Gegenwart von Ammoniak als Lösungsmittel wie oben beschrieben mit Festbettkatalysatoren durch, wobei man nach Desaktivierung des Katalysators, d. h. Absinken von Umsatz, bezogen auf ADN,und/oder Selektivität, bezogen auf ACN, unter einen definierten Wert, zunächst den Zulauf an Adipodinitril und Ammoniak abgestellt, anschließend die Temperatur auf 200 bis 250°C bringt und dann den Katalysator fünf bis sechs Stunden mit von 200 bis 800, bevorzugt von 500 bis 700, insbesondere 600 l Wasserstoff/l Kat. x h behandelt. Danach kühlt man üblicherweise auf Reaktionstemperatur ab und setzt die Hydrierung fort.

Vor Beginn der Regenerierung entfernt man bevorzugt den noch im Reaktor enthaltenen Hydrieraustrag. Des weiteren kann es vorteilhaft sein, insbesondere, wenn man die Behandlung von ADN mit Wasserstoff in Suspension durchführt, den Katalysator vor der eigentlichen Regenerierung, d.h. nach Unterbrechung der Behandlung von ADN mit Wasserstoff (Stufe (a)) und vor der Behandlung mit Wasserstoff (Stufe (b)), mit dem im System enthaltenen Lösungsmittel, insbesondere flüssigem Ammoniak, zu waschen. Die Temperatur beim Waschen wählt man üblicherweise im Bereich von 20 bis 200°C, insbesondere von 20 bis 100°C. Für das Waschen ist in der Regel ein Zeitraum von 2 bis 24 Stunden vorteilhaft.

Nach bisherigen Beobachtungen kann man die Regenerierung zu jedem beliebigen Zeitpunkt vornehmen. Wirtschaftlich betrachtet erscheint eine Regenerierung dann sinnvoll zu sein, wenn der Umsatz, bezogen auf ADN, und/oder die Selektivität, bezogen auf ACN, mehr als 10%, bezogen auf den Anfangswert, abgesunken ist.

Die Regenerierung der Katalysatoren führt man erfindungsgemäß bei Temperaturen im Bereich von 180 bis 270°C, insbesondere von 200 bis 250°C durch, wobei man den Wasserstoffdruck im Bereich von 0,1 bis 30 MPa, bevorzugt 0,1 bis 20 MPa und die Behandlungszeit im Bereich von 2 bis 48 h, bevorzugt von 2 bis 24 h,wählt. Bei kontinuierlicher Fahrweise wählt man die Wasserstoffmenge üblicherweise im Bereich von 100 bis 1500, vorzugsweise von 200 bis 1000 l Wasserstoff/l Reaktorvolumen x Stunde.

Mit Hilfe des erfindungsgemäßen Verfahrens ist es möglich, die Katalysatorstandzeit und die Raum-Zeit-Ausbeute von Nickel-Katalysatoren bei der Hydrierung von Adipodinitril zu 6-Aminocapronitril und Hexamethylendiamin (Zwischenprodukte bei der Nylon 6- und Nylon 66-Herstellung) deutlich zu verbessern.

### Beispiele

### Beispiel 1 (Suspensionshydrierung)

Reaktor: 250 ml Autoklav mit Probenahmeschleuse (Werkstoff HC 4), die Durchmischung erfolgte mit Scheibenrührer.

Ansatz: jeweils 48 g ADN, 5,6 g Raney-Nickel (BASF, H 1-50, wasserfeucht) .

Raney-Nickel wurde unter Schutzgas (Argon) in einen Autoklaven eingefüllt. Anschließend wurde der Autoklav verschlossen und 150 ml fl.NH₃ aufgepreßt. Nach kurzem Rühren wurde die Hauptmenge Ammoniak über ein Steigrohr mit Fritte aus dem Reaktor gedrückt. Dieser Vorgang wurde sechsmal mit je 50 ml fl. Ammoniak wiederholt, um wasserfreies Raney-Nickel als repräsentativen Startkatalysator zu erhalten (hold up an Ammoniak ca. 100 ml). Darauf wurde das System auf 80°C aufgeheizt, 48 g Adipinsäuredinitril wurden zudosiert und der Druck mit Wasserstoff auf 7 MPa erhöht. Aus der Flüssigphase wurden katalysatorfreie Proben durch die Probenahmeschleuse nach 20, 45, 90, 135, 180, und 225 min entnommen.

Nach 225 min wurde die Temperatur im Reaktor auf 25 °C gesenkt und die katalysatorfreie Reaktionsmischung entnommen. Der im Reaktor verbliebene Katalysator wurde, wie beim Waschvorgang vor dem ersten Einsatz beschrieben, sechsmal mit je 50 ml flüssigem Ammoniak bei Raumtemperatur gespült. Für den folgenden Versuch wurde dann das System wieder auf 80°C aufgeheizt und die Reaktanten erneut zudosiert. Die Versuche mit Probennahme und Waschvorgang wurden mehrfach wiederholt.

In Tabelle 1 wird der Umsatz des Adipinsäuredinitrils und die Selektivität zu 6-Aminocapronitril angegeben wie sie sich aus den GC-Daten nach jeweils 225 Minuten Hydrierzeit ergaben. Außer ACN entstand fast ausschließlich Hexamethylendiamin.

**Tabelle 1**

| Versuch | Umsatz ADN | Selektivität ACN |
|---|---|---|
| 1 | 84,2 | 59,6 |
| 2 | 49,6 | 70,4 |
| 3 | 43,4 | 64,7 |
| 4 | 37,3 | 70,1 |
| 5 | 30,6 | 75,2 |
| 6 | 29,2 | 75,2 |
| 7 | 26,3 | 77,2 |
| 8 | 24,2 | 80,0 |
| 9 | 17,3 | 79,5 |
| 10 | 16,2 | 85,0 |
| 11 | 13,2 | 81,6 |
| 12 | 9,0 | 86,8 |
| 13 | 7,4 | 95,3 |
| 14 | 6,0 | 85,5 |
| 15 | 5,3 | 84,6 |
| 16 | 5,4 | 87,2 |
| 17 | 4,9 | 90,3 |
| 18 | 5,8 | 88,2 |

Nach dem 18. Versuch wurde der desaktivierte Katalysator, nach Entfernung des Hydriergemischs sechsmal mit flüssigem Ammoniak gespült. Daraufhin wurde der Ammoniak vollständig entspannt und komplett mit Argon aus dem Reaktor verdrängt. Danach wurde der Reaktor auf 100°C aufgeheizt und nochmals mit Argon gespült. Anschließend wurde das Argon durch Spülung mit Wasserstoff verdrängt. Der Reaktor wurde dann auf 250°C aufgeheizt und der Druck mit Wasserstoff auf 10 MPa eingestellt. 5 Stunden wurde der Reaktor bei 250°C belassen. Anschließend wurde der Autoklav auf Raumtemperatur abgekühlt, die Gasphase völlig entspannt und der nächste Versuchsblock begonnen.

**Tabelle 2 -**

| Versuch nach Regenerierung des Katalysators | | |
|---|---|---|
| Versuch | Umsatz des ADN | Selektivität zu ACN |
| 19 | 54,9 | 80,5 |

Durch die Regenerierung mit Wasserstoff konnte der Umsatz, bezogen auf ADN, von 5,8% auf 54,9 % angehoben werden.

### Beispiel 2 (Festbetthydrierung in der Flüssigphase):

### Katalysatorherstellung

2,5 kg einer NiAl-Legierung (Fa. BASF, H1-55) wurden bei 80°C mit Stearinsäure getränkt. Nach dem Zerkleinern der abgekühlten und erstarrten Masse wurde das erhaltene Pulver zu Tabletten (3 mm Höhe, 3 mm Durchmesser) gepreßt. Die so erhaltenen Tabletten wurden dann bei 900°C 2 h calziniert. Die Aktivierung der Tabletten wurde mit Natronlauge durchgeführt. Dazu wurden 2,4 kg der Tabletten in 5,7 l Wasser vorgelegt und unter starkem Rühren wurden anschließend insgesamt 1,44 kg NaOH-Plätzchen portionsweise zugegeben. Nach Beendigung der Zugabe wurden bei 90°C weitere 24 h gerührt. Nach Abkühlen wurden die aktivierten Tabletten mit Wasser gewaschen bis das Waschwasser pH-neutral war.

Die aktivierten Katalysator-Tabletten wurden unter Wasser in den Reaktor eingebaut und mit Ammoniak gespült.

### Hydrierung:

Pro Stunde wurden 370 g Adipinsäuredinitril und 1,1 kg Ammoniak mit 500 1 Wasserstoff in Sumpffahrweise über einen Rohrreaktor (befüllt mit 740 ml Katalysator; Länge = 1800 mm, Durchmesser 30 5 mm) geleitet. Die Reaktortemperatur betrug 50°C, der Druck 20 MPa. Der Reaktionsaustrag wurde gaschromatographisch analysiert. Nach einer Anfahrphase wurde ein Adiponitril-Umsatz von 45% erhalten, der während einer Versuchsdauer von 280 Stunden auf 20% abfiel. Die Aminocapronitril-Selektivität stieg von anfänglich 80 auf 90%.

Anschließend wurde der Dinitril- und Ammoniak-Zulauf abgestellt und der Katalysator im Reaktor 5 Stunden bei 200°C und einem Wasserstoffdruck von 20 MPa (bei 500 l/h Wasserstoff) regene5 riert. Nach erneutem Anfahren unter gleichen Bedingungen (s. o.) stieg der Umsatz auf 45% (bei einer ACN-Selektivität von 80%), d. h. die Anfangsaktivität des Katalysators wurde erreicht.

## Patentansprüche

1. Verfahren zur gleichzeitigen Herstellung von 6-Aminocapronitril ("ACN") und Hexamethylendiamin ("HMD") durch Behandlung von Adipodinitril ("ADN") mit Wasserstoff in Gegenwart eines Nickel-haltigen Katalysators bei Temperaturen von nicht unterhalb der Raumtemperatur und erhöhtem Wasserstoff-Partialdruck in Suspension oder in einem Festbettreaktor in Riesel- oder Sumpffahrweise in Gegenwart eines Lösungsmittels, **dadurch gekennzeichnet, daß** man nach Absinken des Umsatzes, bezogen auf ADN, und/oder der Selektivität, bezogen auf ACN, unter einen definierten Wert,
(a) die Behandlung von ADN mit Wasserstoff unterbricht, indem man die Zufuhr von ADN und dem gewünschtenfalls vorhandenen Lösungsmittel stoppt,
(b) den Katalysator bei einer Temperatur im Bereich von 180 bis 270°C mit Wasserstoff behandelt, wobei man den Wasserstoffdruck im Bereich von 0,1 bis 30 MPa und die Behandlungszeit im Bereich von 2 bis 48h wählt, und
(c) anschließend die Hydrierung von ADN mit dem nach Stufe (b) behandelten Katalysator fortsetzt.

2. Verfahren nach Anspruch 1, **dadurch gekennzeichnet, daß** man die Behandlung von ADN mit Wasserstoff in Suspension bei einer Temperatur im Bereich von 40 bis 150°C und einem Druck im Bereich von 2 bis 20 MPa durchführt.

3. Verfahren nach Anspruch 1, **dadurch gekennzeichnet, daß** man die Behandlung von ADN mit Wasserstoff in einem Festbettreaktor in Riesel- oder Sumpffahrweise bei einer Temperatur im Bereich von 20 bis 150°C und einem Druck im Bereich von 2 i bis 30 MPa durchführt.

4. Verfahren nach Anspruch 2, **dadurch gekennzeichnet, daß** man nach Unterbrechung der Behandlung von ADN mit Wasserstoff (Stufe (a)) den Katalysator vor der Behandlung mit Wasserstoff (Stufe (b)) mit flüssigem Ammoniak spült und den Ammoniak im Anschluß an das Spülen mit einem Inertgas verdrängt.

## Claims

1. A process for the coproduction of 6-aminocapronitrile (ACN) and hexamethylenediamine (HMD) by treatment of adiponitrile (ADN) with hydrogen in the presence of a nickel-containing catalyst at temperatures not below room temperature and elevated hydrogen partial pressure in suspension or in a fixed-bed reactor in a downflow or upflow process in the presence of a solvent, which comprises, after the conversion based on ADN and/or the selectivity based on ACN has or have dropped below a defined value
(a) interrupting the treatment of ADN with hydrogen by stopping the feed of ADN and of the solvent, if used,
(b) treating the catalyst at from 180 to 270°C with hydrogen using a hydrogen pressure within the range from 0.1 to 30 MPa and a treatment time within the range from 2 to 48 h, and
(c) then continuing the hydrogenation of ADN with the treated catalyst of stage (b).

2. A process as claimed in claim 1, wherein the treatment of ADN with hydrogen is carried out in suspension at a temperature within the range from 40 to 150°C and a pressure within the range from 2 to 20 MPa.

3. A process as claimed in claim 1, wherein the treatment of ADN with hydrogen is carried out in a fixed-bed reactor in a downflow or upflow process at a temperature within the range from 20 to 150°C and a pressure within the range from 2 to 30 MPa.

4. A process as claimed in claim 2, wherein, following interruption of the treatment of ADN with hydrogen (stage (a)) and before the treatment of the catalyst with hydrogen (stage (b)), the catalyst is rinsed with liquid ammonia and, after the rinse, the ammonia is displaced with an inert gas.

## Revendications

1. Procédé de préparation simultanée de 6-aminocapronitrile ("ACN") et d'hexaméthylène-diamine ("HMD") par traitement d'adiponitrile ("ADN") par de l'hydrogène, en présence d'un catalyseur au nickel, à des températures non inférieures à la température ambiante et sous pression partielle d'hydrogène élevée en mode de suspension ou dans un réacteur à lit fixe, en mode à écoulement ou de fond de cuve en présence d'un solvant, **caractérisé en ce que**, lorsque le taux de conversion, par rapport à l'ADN, et/ou la sélectivité par rapport à l'ACN, tombe(nt) au-dessous d'une valeur déterminée,
(a) on interrompt le traitement de l'ADN à l'hydrogène, en arrêtant l'amenée d'ADN et du solvant éventuellement présent,
(b) on traite le catalyseur par de l'hydrogène à une température de l'ordre de 180 à 270°C, en choisissant la pression d'hydrogène dans la plage de 0,1 à 30 MPa et le temps de traitement dans la plage de 2 à 48 heures, et
(c) on reprend ensuite l'hydrogénation de l'ADN avec le catalyseur traité à l'étape (b).

2. Procédé selon la revendication 1, **caractérisé en ce que** l'on entreprend le traitement de l'ADN par de l'hydrogène en mode de suspension à une température de l'ordre de 40 à 150°C et une pression de l'ordre de 2 à 20 MPa.

3. Procédé selon la revendication 1, **caractérisé en ce que** l'on entreprend le traitement de l'ADN par de l'hydrogène dans un réacteur à lit fixe, en mode à écoulement ou de fond de cuve, à une température de l'ordre de 20 à 150°C et une pression de l'ordre de 2 à 30 MPa.

4. Procédé selon la revendication 2, **caractérisé en ce qu'**après l'interruption du traitement de l'ADN par de l'hydrogène (étape (a)), le catalyseur est rincé à l'ammoniac liquide avant le traitement à l'hydrogène (étape (b)) et que l'ammoniac est chassé, après le rinçage, au moyen d'un gaz inerte.
